# FASCICULE DE BREVET EUROPEEN

(11) **EP 2 494 495 B1**
(45) Date de publication et mention de la délivrance du brevet: **21.12.2016**
(21) Numéro de dépôt: 10768501.8
(22) Date de dépôt: 25.10.2010
(51) Int. Cl.: G06K 9/00, G06K 9/20, A61B 5/00

(54) **PROCÉDÉ ET DISPOSITIF POUR ASSERVIR L'ACTIVATION D'UN ENSEMBLE D'ÉMETTEURS INFRAROUGES D'UN CAPTEUR DE RÉSEAUX VEINEUX À LA PRÉSENCE D'UN CORPS VIVANT**
VERFAHREN UND VORRICHTUNG ZUR REGULIERUNG DER AKTIVIERUNG EINER REIHE AN INFRAROTEMITTERN EINES SENSORS EINES VENENNETZES IN GEGENWART EINES LEBENDEN KÖRPERS
METHOD AND DEVICE FOR SLAVING THE ACTIVATION OF A SET OF INFRARED EMITTERS OF A SENSOR OF VENOUS NETWORKS TO THE PRESENCE OF A LIVING BODY

(30) Priorité: 26.10.2009 FR 0957479
(43) Date de publication de la demande: 05.09.2012
(73) Titulaire: MORPHO, 92130 Issy-Les-Moulineaux (FR)
(72) Inventeur: MORIN, Aurélie, F-75015 Paris (FR); DARBOIS, Matthieu, F-75015 Paris (FR); GOUDON, Olivier, F-75015 Paris (FR); BALZAC, Yannick, F-75015 Paris (FR)
(74) Mandataire: Le Guen-Maillet
(86) Numéro de dépôt international: PCT/EP2010/066071
(87) Numéro de publication internationale: WO 2011/051230

(56) Documents cités:
- EP-A2- 2 172 911
- US-A- 3 970 990
- US-A- 4 547 898
- US-A1- 2005 047 632
- US-A1- 2009 060 301
- US-A1- 2009 245 591
- US-B1- 7 620 212
- M. Watanabe: "Palm Vein Authentication" In: N. Ratha and V. Govindaraju (Eds.): "Advances in Biometrics", 2008, Springer, XP002615593, pages 75-80, third paragraph; page 78

## Description

Procédé et dispositif pour asservir l'activation d'un ensemble d'émetteurs infrarouges d'un capteur de réseaux veineux à la présence d'un corps vivant.

La présente invention concerne un procédé et dispositif pour asservir l'activation d'un ensemble d'émetteurs infrarouges d'un capteur de réseaux veineux à la présence d'un corps vivant.

Un capteur de réseaux veineux d'un corps vivant tel que celui d'un doigt, d'une main, d'un orteil, etc. est très utilisé, par exemple pour le contrôle d'accès à un site ou une machine basé sur des données biométriques. Par exemple, dans le cas d'un système de contrôle d'accès par empreinte digitale, un capteur de réseaux veineux est associé à un capteur d'empreinte digitale pour augmenter la fiabilité du système de contrôle d'accès car il permet d'assurer que l'empreinte digitale acquise est bien issue d'un doigt vivant et d'authentifier un utilisateur par comparaison de son empreinte digitale et du réseau veineux de son doigt avec des données biométriques préalablement enregistrées.

Un capteur de réseaux veineux comporte une source infrarouge qui éclaire un moyen d'acquisition d'images du capteur tel que par exemple une caméra CCD.

Cette source infrarouge est réalisée par un ensemble d'émetteurs infrarouges qui sont habituellement positionnés les uns par rapport aux autres selon des lignes et/ou des colonnes. On parle alors de barrettes ou de matrices d'émetteurs infrarouges.

Lorsqu'un corps vivant est placé entre la source infrarouge et le moyen d'acquisition d'images, certains rayons du flux lumineux frappent directement le moyen d'acquisition d'images et d'autres frappent ce moyen une fois qu'ils ont traversé le corps vivant. L'image ainsi acquise est alors traitée numériquement pour que le réseau veineux du corps vivant apparaisse sur cette image. L'authentification d'un utilisateur peut alors être réalisée par comparaison du réseau veineux ainsi acquis et du réseau veineux qu'il a préalablement enregistré.

Si le principe d'un capteur de réseaux veineux est simple, sa mise en oeuvre pose le problème de surexposition de l'image acquise. En effet, les rayons qui frappent directement le moyen d'acquisition d'images induisent une intensité des pixels de l'image qui est importante comparée à celle induite par les rayons qui traversent le corps vivant. Cette différence d'intensité provoque des artéfacts sur l'image acquise, tels qu'un halo lumineux ou des surintensités locales, surtout aux bords du corps vivant. Ces artéfacts perturbent les traitements qui sont appliqués à l'image acquise pour faire apparaître le réseau veineux.

Pour améliorer la qualité de l'acquisition du réseau veineux, les capteurs de réseaux veineux comportent des moyens pour asservir l'activation de l'ensemble des émetteurs infrarouges à la présence d'un corps vivant. Ainsi, lorsqu'une partie du corps vivant est présente entre l'un des émetteurs infrarouges et le moyen d'acquisition d'images, cet émetteur infrarouge est activé, et lorsque un émetteur infrarouge éclaire directement le moyen d'acquisition, alors cet émetteur infrarouge est désactivé.

Il est connu que de tels moyens d'asservissement sont mis en oeuvre par traitement d'images.

En particulier, le brevet US2002048014A1 décrit un procédé d'asservissement qui consiste, dans un premier temps, à activer faiblement tous les émetteurs infrarouges de l'ensemble lorsqu'un corps vivant est présent. Une image est ensuite acquise par le moyen d'acquisition d'images, et la position du corps vivant dans l'image est détectée par traitement de l'image ainsi acquise. L'intensité des pixels de l'image est alors considérée et comparée à une valeur de référence. Lorsque l'intensité d'un pixel de l'image est inférieure à cette valeur de référence, l'activation de l'émetteur infrarouge qui a induit l'intensité de ce pixel est augmentée de manière à ce que le réseau veineux puisse être extrait à partir d'une prochaine acquisition d'image. A l'opposé, lorsque l'intensité d'un pixel de l'image est supérieure à cette valeur de référence, l'émetteur infrarouge qui a induit l'intensité de ce pixel est désactivé.

Ainsi, par analyse des intensités d'une image acquise lorsque les émetteurs infrarouges sont faiblement activés, il est déterminé quels sont ceux de ces émetteurs infrarouges qui doivent être activés fortement et ceux qui doivent être désactivés. Lorsque le corps vivant se déplace, une nouvelle image est alors acquise pour déterminer à nouveau ceux des émetteurs infrarouges qui doivent être activés fortement et ceux qui doivent être désactivés. L'activation (et la désactivation) des émetteurs infrarouges est ainsi asservie à la présence d'un corps vivant entre l'ensemble d'émetteurs infrarouges et le moyen d'acquisition d'images.

Une fois que le doigt est positionné de manière stable, c'est-à-dire que les émetteurs infrarouges restent dans un état stable (fortement activés ou désactivés) pendant une durée de temps, une nouvelle image est acquise et le réseau veineux est alors extrait à partir de cette nouvelle image.

Un tel procédé d'asservissement par analyse des intensités d'une image acquise présente plusieurs inconvénients.

Tout d'abord, il est nécessaire de calibrer le moyen d'acquisition d'images pour connaître les positions des émetteurs infrarouges dans le plan de l'image. En d'autres termes, ce procédé d'asservissement requiert que l'on connaisse préalablement l'émetteur infrarouge qui induit l'intensité de chaque pixel de l'image. Cette contrainte de mise en oeuvre est d'autant plus amplifiée lorsque le capteur est calibré lors de sa fabrication car les positions relatives de l'ensemble d'émetteurs infrarouges et du moyen d'acquisition d'images doivent rester fixes pour que le procédé d'asservissement continue de fonctionner correctement. Ceci implique soit des contraintes mécaniques au niveau du capteur de réseaux veineux, soit de prévoir une phase de calibration avant tout usage du capteur.

Ensuite, ce procédé d'asservissement ne sera pas opérationnel lorsque le corps vivant se déplace rapidement. En effet, pour que le réseau veineux puisse être extrait d'une image acquise, il faut au préalable qu'une autre image soit acquise pour l'activation/désactivation des émetteurs infrarouges. Le procédé d'asservissement par analyse des intensités d'une image acquise n'est donc pas adapté pour l'acquisition à la volée d'un réseau veineux car un délai de traitement incompressible et systématique se produit entre la position courante du corps vivant et l'acquisition de l'image à partir de laquelle le réseau veineux sera extrait.

Le problème résolu par la présente invention est de remédier aux inconvénients des procédés d'asservissement de l'état de la technique.

Le document US-A-2009/060301 divulgue un capteur d'image de réseau veineux avec un traitement particulier des pixels.

Le document EP-A-2 172 911 divulgue un capteur d'image qui comporte deux détecteurs de présence et deux émetteurs infrarouges qui sont disposés l'un en face de l'autre.

A cet effet, la présente invention concerne un procédé pour asservir l'activation d'un ensemble d'émetteurs infrarouges d'un capteur de réseaux veineux à la présence d'un corps vivant entre cet ensemble et un moyen d'acquisition d'images du capteur. Le procédé est caractérisé en ce que chaque émetteur infrarouge est activé si la présence d'une partie du corps vivant est détectée par au moins un détecteur de présence qui lui est associé et chaque émetteur infrarouge est désactivé tant que la présence d'une partie du corps vivant n'est pas détectée.

L'association d'au moins un détecteur de présence à chacun des émetteurs infrarouges de l'ensemble permet de désolidariser le procédé d'asservissement du moyen d'acquisition d'images et donc d'éviter de devoir acquérir une image pour décider de l'activation ou désactivation de chaque émetteur infrarouge.

Un capteur de réseaux veineux mettant en oeuvre un tel procédé peut alors être utilisé pour l'acquisition à la volée d'un réseau veineux car le délai inhérent au procédé d'asservissement de l'état de la technique n'existe plus.

De plus, cette désolidarisation du procédé et du moyen d'acquisition évite la mise en oeuvre d'une phase préalable de calibration, facilitant ainsi la fabrication du capteur ainsi que son utilisation.

La présente invention concerne également un ensemble d'émetteurs infrarouges prévu pour éclairer un moyen d'acquisition d'un capteur de réseau veineux. Cet ensemble est caractérisé en ce qu'il comporte au moins un détecteur de présence associé à chaque émetteur infrarouge.

Selon un mode de réalisation, quatre détecteurs de présence sont associés à chaque émetteur infrarouge de l'ensemble, chacun de ces quatre détecteurs de présence étant positionné à un point cardinal de chaque émetteur infrarouge.

Ce positionnement particulier des détecteurs de présence évite que le mouvement du corps vivant ne soit guidé pour être détecté.

Selon un mode de réalisation, chaque émetteur infrarouge et chaque détecteur de présence de l'ensemble sont positionnés selon au moins une ligne et/ou selon au moins une colonne, un émetteur infrarouge alternant avec un détecteur de présence sur chaque ligne et/ou sur chaque colonne.

La présente invention concerne, de plus, un dispositif pour asservir l'activation d'un ensemble d'émetteurs infrarouges ci-dessus et qui comporte des moyens pour activer un émetteur infrarouge si la présence d'une partie du corps vivant est détectée par au moins un détecteur de présence qui est associé à cet émetteur infrarouge, et pour désactiver un émetteur infrarouge tant que la présence d'une partie du corps vivant n'est pas détectée.

Enfin, la présente invention concerne un capteur de réseaux veineux d'une partie d'un corps vivant comportant un ensemble d'émetteurs infrarouges et un dispositif ci-dessus.

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels:
La Fig. 1 représente un schéma d'un capteur de réseau veineux,
Les Fig. 2a-2d représentent des différents exemples d'associations de détecteurs de présence avec un émetteur infrarouge,
La Fig. 3 représente un exemple de positionnement des émetteurs infrarouges,
La Fig. 4a représente un autre exemple de positionnement des émetteurs infrarouges,
La Fig. 4b représente un mode préféré de positionnement des émetteurs infrarouges,
La Fig. 5 représente un mode de réalisation du positionnement d'une source de lumière dans le capteur de réseau veineux, et
La Fig. 6 représente un autre mode de réalisation du positionnement d'une source de lumière dans le capteur de réseau veineux.

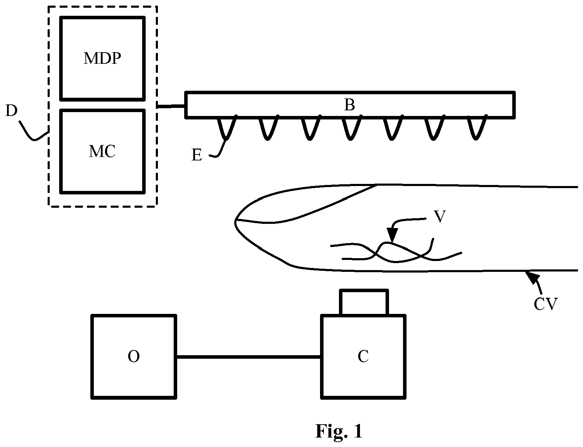

A la Fig. 1 est représenté schématiquement un exemple d'un capteur de réseaux veineux prévu pour acquérir une image du réseau veineux V d'un doigt CV. On comprendra que ce capteur n'est qu'un exemple de réalisation qui peut être adapté, notamment, pour l'acquisition de réseau veineux d'autres corps vivants tels que une main, un orteil etc.

Le capteur comporte un ensemble B d'émetteurs infrarouges E, tels que par exemple des diodes électroluminescentes (en anglais Light-Emittting Diode) ou des diodes électroluminescentes organiques (en anglais Organic Light-Emittting Diode), un moyen d'acquisition d'images C, tel qu'une caméra CCD, positionné en face de l'ensemble B de sorte que les émetteurs infrarouges E éclairent ce moyen C, et des moyens O de traitements d'images qui sont reliés au moyen d'acquisition C de manière à, d'une part, obtenir une image acquise par le moyen C et, d'autre part, faire apparaître dans cette image le réseau veineux V du doigt CV par application d'un traitement sur l'image acquise tel que, par exemple, celui décrit dans la partie introductive.

Selon une caractéristique de la présente invention, au moins un détecteur de présence DP est associé à chaque émetteur infrarouge E de l'ensemble B. La fonction de ce détecteur de présence DP est de déterminer si une partie du doigt est présente entre cet émetteur infrarouge et le moyen C.

Plusieurs types de détecteurs de présence de l'état de la technique peuvent être utilisés. La contrainte imposée est qu'ils doivent donner une indication binaire relative à la présence ou non de la partie du doigt CV.

Selon un mode de réalisation, au moins un des détecteurs de présence DP associé à au moins un émetteur infrarouge E de l'ensemble B est un capteur ultrason. Les capteurs à ultrasons, appelés également télémètres à ultrasons, font partie des capteurs qui permettent de mesurer des distances sans contact. Le télémètre à ultrasons est basé sur la mesure du temps écoulé entre l'émission d'une onde ultrasonore et la réception d'un écho. Plus précisément, lorsque l'onde ultrasonore est émise, elle se propage à la vitesse du son, dans l'air environnant à 342 m/sec. Dès qu'un obstacle est rencontré, l'écho revient vers le transducteur du télémètre qui calcule alors le temps écoulé entre l'émission et la réception de l'onde et en déduit alors la distance entre lui et l'obstacle. Ainsi, en l'absence d'une partie du doigt CV, le capteur ultrason détermine une première distance entre lui et un obstacle tel que par exemple le moyen d'acquisition C ou un support sur lequel est posé le capteur de réseau veineux, et lorsqu'une partie du doigt CV se trouve à proximité d'un capteur ultrason, une autre distance est déterminée. La présence d'une partie du doigt est alors détectée si cette autre distance est inférieure à la première distance.

Selon un autre mode de réalisation, au moins un des détecteurs de présence associé à au moins un émetteur infrarouge E de l'ensemble B est un détecteur infrarouge. Ce type de détecteur détecte le mouvement d'un corps dont la température est proche de celle du corps humain, en l'occurrence une partie du doigt CV.

Selon un autre mode de réalisation, au moins un des détecteurs de présence associé à au moins un émetteur infrarouge E de l'ensemble B est un télémètre laser.

Selon un autre mode de réalisation, au moins un des détecteurs de présence DP associé à au moins un émetteur infrarouge E de l'ensemble E est un interrupteur réagissant à un flux lumineux, tel que par exemple une photodiode, un phototransistor. Une photodiode ou phototransistor reste dans un état « ouvert » tant qu'il n'est pas éclairé par un flux lumineux et bascule vers un état « fermé » dès qu'il est éclairé.

On peut noter que la présente invention n'est pas conditionnée à l'utilisation exclusive de l'un ou l'autre des détecteurs de présence DP ci-dessus. Plusieurs types de détecteurs de présence peuvent être associés à un même émetteur infrarouge et/ou un même type de détecteur de présence peut être associé à un même émetteur infrarouge et/ou le type de détecteur de présence diffère d'un émetteur infrarouge à l'autre.

Le nombre de détecteurs de présence par émetteur infrarouge et leurs positionnements relatifs autour d'un même émetteur infrarouge sont deux paramètres qui conditionnent la logique de détection de la présence d'une partie du doigt entre cet émetteur infrarouge et le moyen C.

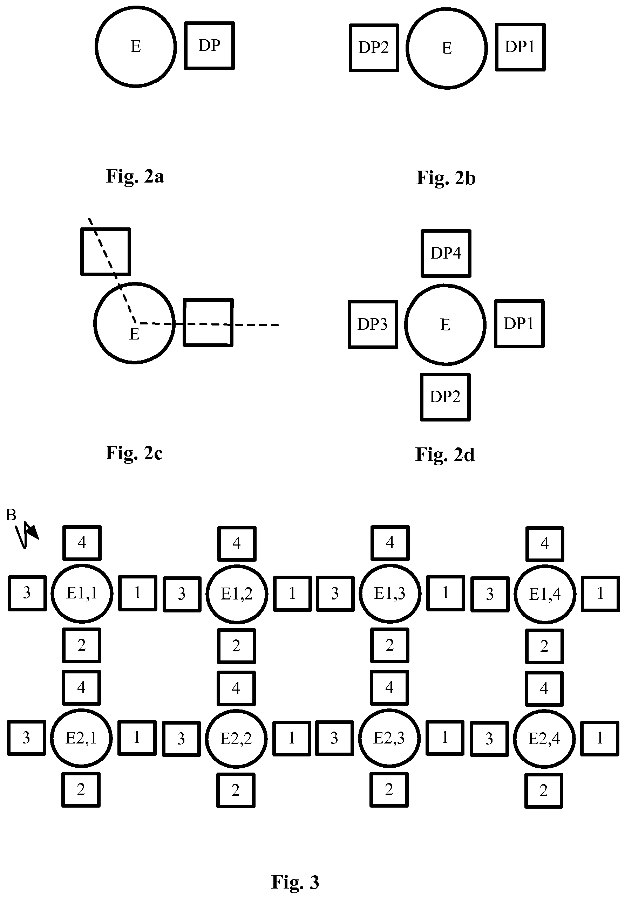

Les Fig. 2a-2d présentent différents exemples d'associations de détecteurs de présence avec un émetteur infrarouge.

Un seul détecteur de présence DP peut être associé à un émetteur infrarouge (Fig. 2a).

Ce détecteur de présence DP peut être intégré à l'émetteur infrarouge.

Ce détecteur de présence DP peut également être positionné autour de l'émetteur infrarouge, par exemple selon l'un quelconque des points cardinaux de l'émetteur infrarouge, c'est-à-dire en haut, en bas, à gauche ou à droite.

Il n'y a aucune logique particulière dans ces deux derniers cas car le détecteur de présence DP indique directement la présence d'une partie du doigt.

Deux détecteurs de présence DP1 et DP2 peuvent être associés à un même émetteur infrarouge E. Ces deux détecteurs de présence peuvent être alignés, tels que illustrés à la Fig. 2b, ou former un angle particulier entre eux, tel que illustré à la Fig. 2c. La logique de détection peut alors être soit de considérer que le doigt est présent dès que l'un des deux détecteurs de présence DP1 ou DP2 indique cette présence, soit de ne le considérer que si les deux détecteurs de présence DP1 et DP2 l'indiquent.

Plus de deux détecteurs de présence DP peuvent être associés à un même émetteur infrarouge E et une logique de détection particulière peut être considérée sans toutefois sortir du cadre de la présente invention.

Selon un mode préféré illustré à la Fig. 2d, quatre détecteurs de présence DP1,..., DP4 sont associés à un émetteur infrarouge E. Chacun de ces quatre détecteurs de présence est positionné à un point cardinal de l'émetteur infrarouge. La logique de détection peut alors être de considérer que le doigt est présent soit dès que l'un des quatre détecteurs de présence indique la présence d'une partie du doigt, soit dès que deux des quatre détecteurs de présence l'indiquent, soit dès que trois des quatre détecteurs de présence l'indiquent, soit uniquement lorsque les quatre détecteurs de présence l'indiquent.

L'ensemble B d'émetteurs infrarouges comporte plusieurs émetteurs infrarouges E qui peuvent être positionnés les uns par rapport aux autres d'une manière particulière sans pour cela sortir du cadre de la présente invention.

Selon un mode de réalisation illustré par la Fig. 3, les émetteurs infrarouges Ei,j (i=1 à L et j=1 à C) sont positionnés selon L lignes et C colonnes de manière à former une matrice de L lignes et C colonnes. On entend par là que C ou L peuvent être égales à l'unité. On parle alors de barrette. Selon l'exemple de la Fig. 3, l'ensemble B comporte 2 lignes et 4 colonnes et quatre détecteurs de présence, notés 1,2,3 et 4 sont associés à chaque émetteur infrarouge Ei,j.

Les dimensions de l'ensemble B de la Fig. 3 sont importantes du fait du nombre important de détecteurs de présence. Il est alors avantageux d'alterner par ligne et/ou par colonne un émetteur infrarouge Ei,j et un détecteur de présence. Le nombre de détecteurs de présence est ainsi diminué, ce qui induit, d'une part, une diminution du coût de fabrication de l'ensemble B et, d'autre part, une diminution de ses dimensions.

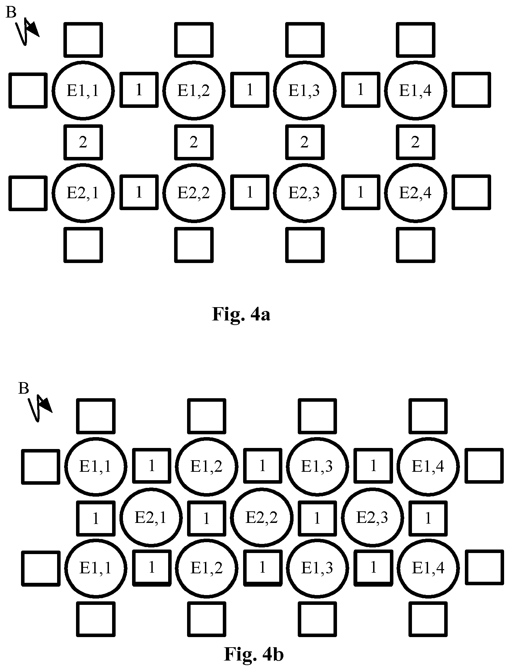

Selon l'exemple de la Fig. 4a, cette alternance est réalisée selon chaque ligne et chaque colonne. Ainsi, les détecteurs de présence marqués 1, respectivement 2, sont associés à deux émetteurs infrarouges de deux colonnes consécutives et de la même ligne, respectivement de deux lignes consécutives et de la même colonne. Les autres détecteurs de présence (sans aucune marque sur la Fig. 4a) sont associés à un seul émetteur infrarouge.

Selon le mode préféré de la Fig. 4b, cette alternance est aussi réalisée selon chaque ligne et chaque colonne mais les détecteurs de présence marqués 1, sont associés, cette fois-ci, à trois émetteurs infrarouges. Les autres détecteurs de présence (sans aucune marque sur la Fig. 4b) sont associés à un seul émetteur infrarouge.

Ce mode préféré optimise les dimensions de l'ensemble B.

La logique de détection préférée, qui est mise en oeuvre pour l'ensemble B décrit en relation avec les Figs. 3, 4a ou 4b, est de considérer alors la présence d'une partie du doigt CV entre un émetteur infrarouge et le moyen C lorsque les quatre détecteurs de présence DP qui lui sont associés indiquent la présence d'une partie du doigt. Cette logique de détection assure que l'émetteur infrarouge, lorsqu'il sera activé, n'éclairera pas directement le moyen C même de manière partielle.

D'autres modes de réalisation sont bien entendu possibles, en réalisant cette alternance uniquement selon les lignes ou les colonnes ou selon certaines lignes et/ou certaines colonnes.

De retour à la Fig. 1, selon une autre caractéristique, le capteur de réseau veineux comporte également un dispositif D pour asservir l'activation des émetteurs infrarouges E à la présence du doigt CV entre l'ensemble B et le moyen C. Le dispositif D comporte des moyens MDP pour vérifier si l'un des détecteurs de présence DP indique la présence d'une partie d'un corps vivant, en l'occurrence d'un doigt. Le dispositif D comporte également des moyens MC pour activer un émetteur infrarouge E de l'ensemble B en mettant en oeuvre une logique de détection, qui comme expliqué en relation avec les Fig. 2a-2d, 3 et 4, est fonction du nombre de détecteurs de présence DP associés à chaque émetteur infrarouge E et de leurs positionnements relatifs autour d'un même émetteur infrarouge.

Les moyens MDP et MC peuvent, par exemple, être mis en oeuvre par un circuit électronique qui peut être formé de portes logiques et/ou inclure, un micro-processeur ou un microcontrôleur.

Selon un mode de réalisation du dispositif D relatif au cas où au moins un des détecteurs de présence DP est un interrupteur réagissant à un flux lumineux, le dispositif D comporte une source lumineuse unique S qui est prévue pour éclairer ledit au moins un détecteur de présence DP.

Selon un mode de réalisation, la source lumineuse S est formée par au moins un des émetteurs infrarouges E de l'ensemble B.

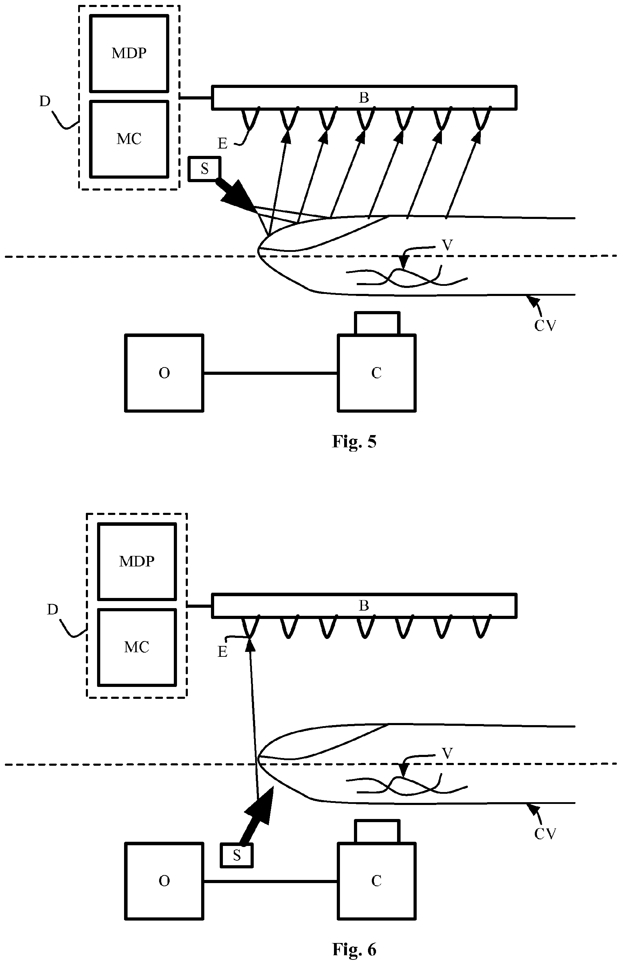

Selon un mode de réalisation du dispositif, la source S est positionnée de manière à ce que son faisceau lumineux, représenté en trait gras sur la Fig. 5, éclaire la région où est susceptible de se trouver le doigt CV. Ainsi, lorsque le doigt CV est présent dans cette région (ou une partie de ce doigt), le faisceau lumineux est réfléchi sur ce doigt et éclaire le ou les détecteurs de présence DP (photodiodes ou phototransistors) associés à chaque émetteur infrarouge lorsqu'une partie du doigt se trouve entre cet émetteur infrarouge et le moyen C. Selon l'exemple de la Fig. 5, tous les détecteurs de présence sont éclairés excepté celui ou ceux associés à l'émetteur infrarouge noté E.

Selon un autre mode de réalisation du dispositif, la source S est positionnée de manière à éclairer les détecteurs de présence DP (photodiodes ou phototransistors) lorsque le doigt est absent et, à ne plus éclairer les détecteurs de présence DP lorsqu'une partie du doigt est présente entre les émetteurs infrarouges qui sont associés à ces détecteurs de présence DP et le moyen C, tel que illustré à la Fig. 6. Selon cet exemple, seuls le ou les détecteurs de présence DP associés à l'émetteur infrarouge E sont encore éclairés en présence du doigt CV.

Ainsi, de manière générale, le dispositif D met en oeuvre un procédé pour asservir l'activation de l'ensemble d'émetteurs infrarouges E à la présence d'un corps vivant, en l'occurrence un doigt, entre cet ensemble et le moyen C.

Au cours de ce procédé, il est vérifié l'état de chaque détecteur de présence DP. Lorsque l'un de ces détecteurs de présence DP indique la présence d'une partie d'un corps vivant, en l'occurrence d'un doigt, chaque émetteur infrarouge Ei,j est activé si la présence d'une partie du doigt est détectée par au moins un détecteur de présence DP qui lui est associé et chaque émetteur infrarouge Ei,j est désactivé tant que la présence d'une partie du doigt n'est pas détectée.

Selon un mode de réalisation du procédé relatif au cas où un seul détecteur de présence DP est associé à chaque émetteur infrarouge Ei,j, l'émetteur infrarouge Ei,j est activé si la présence d'une partie du doigt est détectée par ce capteur de présence DP et cet émetteur Ei,j est désactivé tant que la présence d'une partie du doigt n'est pas détectée par ce détecteur de présence DP.

Selon le mode de réalisation préféré du procédé relatif au cas où plusieurs détecteurs de présence DP sont associés à chaque émetteur infrarouge Ei,j tel que par exemple un cas décrit en relation avec l'une des Figs. 3, 4a ou 4b, dès que l'un des détecteurs de présence DP indique la présence d'une partie d'un doigt, par exemple le détecteur de droite de l'émetteur infrarouge E1,4, le procédé est en attente que d'autres détecteurs de présence DP indiquent la présence de ce doigt. Lorsque les quatre détecteurs de présence DP associés à l'émetteur infrarouge E1,4 indiquent simultanément la présence du doigt, l'émetteur infrarouge E,1,4 est activé et une image du doigt peut alors être acquise par le moyen C. Il en est de même de l'activation des autres émetteurs infrarouges de l'ensemble B. Dès que l'un de ces quatre détecteurs n'indique plus la présence du doigt, l'émetteur infrarouge E,1,4 est désactivé.

Ainsi, dès qu'un émetteur infrarouge de l'ensemble B est activé, une image du doigt peut être acquise.

Dans le cas où la source lumineuse S est formée par au moins un émetteur infrarouge E de l'ensemble B ou lorsque cette source lumineuse S est un émetteur infrarouge, l'état d'activation/désactivation des émetteurs infrarouges de l'ensemble B reste constant pendant la totalité de l'intégration du moyen C, c'est-à-dire pendant l'acquisition d'une image par le moyen C. Ceci afin d'obtenir une image exploitable pour l'extraction du réseau veineux du doigt.

Quoi qu'il en soit, on comprend que le procédé d'asservissement selon la présente invention est très rapide comparé à celui de l'état de la technique. Il est par conséquent très avantageux car il permet que le réseau veineux d'un corps vivant, tel qu'un doigt, puisse être acquis même si ce corps vivant n'est présent qu'un très court instant entre l'ensemble d'émetteurs infrarouges et le moyen C.

## Revendications

1. Procédé pour asservir, à la présence d'un corps humain, l'activation d'un ensemble d'émetteurs infrarouges d'un capteur de réseaux veineux comprenant également un moyen d'acquisition situé en face de l'ensemble d'émetteurs infrarouges,
le procédé comportant une étape de détection de présence du corps vivant,
le procédé étant **caractérisé en ce que** le capteur comprend également un ensemble de détecteurs de présence situé du même côté que les émetteurs infrarouge par rapport au moyen d'acquisition, chaque détecteur de présence étant associé à au moins un émetteur infrarouge, chaque émetteur infrarouge étant associé à au moins un détecteur de présence,
**en ce que** le procédé comporte après l'étape de détection:
- une étape d'activation au cours de laquelle chaque émetteur infrarouge (E) est activé si la présence d'une partie du corps vivant (CV) est détectée par au moins un détecteur de présence (DP) qui lui est associé, et dans le cas contraire :
- une étape de désactivation au cours laquelle ledit émetteur infrarouge (E) est désactivé, et
**en ce que** l'étape de détection est telle que chaque détecteur de présence détermine si une partie du corps vivant est présente entre l'émetteur infrarouge auquel ce détecteur de présence est associé et le moyen d'acquisition.

2. Procédé selon la revendication 1, dans lequel un seul détecteur de présence (DP) étant associé à chaque émetteur infrarouge (E),
- au cours de l'étape d'activation, un émetteur infrarouge (E) est activé si la présence d'une partie du corps vivant est détectée par le détecteur de présence (DP) qui lui est associé, et
- au cours de l'étape de désactivation, un émetteur infrarouge (E) est désactivé tant que la présence d'une partie du corps vivant n'est pas détectée par le détecteur de présence (DP) qui lui est associé.

3. Procédé selon la revendication 1, dans lequel plusieurs détecteurs de présence (DP) sont associés à chaque émetteur infrarouge (E),
- au cours de l'étape d'activation, un émetteur infrarouge (E) est activé lorsque tous les détecteurs de présence (DP) qui lui sont associés indiquent simultanément la présence d'une partie du corps vivant, et
- au cours de l'étape de désactivation, un émetteur infrarouge (E) est désactivé dès que l'un de ces détecteurs de présence (DP) n'indique plus la présence d'une partie du corps vivant.

4. Procédé selon l'une des revendications 1 à 3, dans lequel une image est acquise par le moyen d'acquisition d'images du capteur dès qu'un émetteur infrarouge de l'ensemble est activé.

5. Procédé selon l'une des revendications 1 à 4, dans lequel les étapes d'activation et de désactivation des émetteurs infrarouges (E) ne sont pas mises en oeuvre pendant l'acquisition d'une image par le moyen d'acquisition d'images du capteur.

6. Capteur de réseaux veineux d'une partie d'un corps vivant comportant:
- un ensemble d'émetteurs infrarouges,
- un moyen d'acquisition d'images de ce réseau veineux situé en face de l'ensemble d'émetteurs infrarouges,
et **caractérisé en ce qu'**il comporte en outre:
- un ensemble de détecteurs de présence situé du même côté que les émetteurs infrarouge par rapport au moyen d'acquisition,
chaque détecteur de présence étant associé à au moins un émetteur infrarouge, chaque émetteur infrarouge étant associé à au moins un détecteur de présence, chaque détecteur de présence étant adapté pour déterminer si une partie du corps vivant est présente entre l'émetteur infrarouge auquel ce détecteur de présence est associé et le moyen d'acquisition
- des moyens d'activation d'au moins un émetteur infrarouge, mis en oeuvre si la présence d'une partie du corps vivant (CV) est détectée par au moins un détecteur de présence (DP) qui lui est associé;
- des moyens de désactivation d'au moins un émetteur infrarouge mis en oeuvre pour ledit émetteur infrarouge (E) dans le cas contraire.

7. Capteur selon la revendication 6, **caractérisé en ce que** les émetteurs infrarouges et les détecteurs de présence sont positionnés en ligne et colonne.

8. Capteur selon la revendication 7, **caractérisé en ce que** dans chaque ligne et colonne, un émetteur infrarouge alterne avec un détecteur de présence.

9. Capteur selon la revendication 7, **caractérisé en ce que** quatre détecteurs de présence sont associés avec chaque émetteur infrarouge, et **en ce que** chacun de ces quatre détecteurs est positionné à un point cardinal dudit émetteur infrarouge.

10. Capteur selon l'une des revendications 6 à 9, **caractérisé en ce que** au moins un détecteur de présence de l'ensemble est un interrupteur réagissant à un flux lumineux, et **en ce que** le capteur comporte une source lumineuse unique (S) prévue pour éclairer ledit au moins un interrupteur.

11. Capteur selon la revendication 10, **caractérisé en ce que** la source lumineuse unique (S) est positionnée pour soit éclairer ledit au moins un interrupteur par réflexion sur une partie du corps vivant, soit éclairer ledit au moins un interrupteur en l'absence d'un corps vivant.

## Patentansprüche

1. Steuerverfahren zum Steuern der Aktivierung einer Gruppe von Infrarotmeldern eines Venensystemsensors durch die Anwesenheit eines menschlichen Körpers, das Verfahren zudem umfassend ein, gegenüber der Infrarotmeldergruppe angeordnetes Erfassungsmittel,
wobei das Verfahren einen Schritt umfasst, der darin besteht, die Anwesenheit des lebenden Körpers zu erkennen,
wobei das Verfahren **dadurch gekennzeichnet ist, dass** der Sensor auch eine Gruppe von Anwesenheitsdetektoren umfasst, die sich in Bezug auf das Erfassungsmittel auf derselben Seite wie die Infrarotmelder befinden, wobei jeder Anwesenheitsdetektor wenigstens einem Infrarotmelder und jeder Infrarotmelder wenigstens einem Anwesenheitsdetektor zugeordnet ist,
sowie dadurch, dass das Verfahren nach dem Erkennungsschritt folgende Schritte umfasst:
einen Schritt der Aktivierung, in dessen Verlauf jeder Infrarotmelder (E) aktiviert wird, wenn die Anwesenheit eines Teils des lebenden Körpers (CV) von wenigstens einem, dem Infrarotmelder (E) zugeordneten Anwesenheitsdetektor (DP) erkannt wird und im umgekehrten Fall:
einen Schritt der Deaktivierung, in dessen Verlauf der genannte Infrarotmelder (E) deaktiviert wird,
und dadurch, dass der Erkennungsschritt derart abläuft, dass jeder Anwesenheitsdetektor bestimmt, ob ein Teil des lebenden Körpers zwischen dem Infrarotmelder, dem dieser Anwesenheitsdetektor zugeordnet ist, und dem Erfassungsmittel anwesend ist.

2. Verfahren nach Patentanspruch 1, demzufolge ein einziger Anwesenheitsdetektor (DP) jedem Infrarotmelder (E) zugeordnet ist, wobei
- im Verlauf des Aktivierungsschritts ein Infrarotmelder (E) aktiviert wird, wenn die Anwesenheit eines Teils des lebenden Körpers von dem Anwesenheitsdetektor (DP) detektiert wird, der diesem Infrarotmelder zugeordnet ist, und
- im Verlauf des Deaktivierungsschritts ein Infrarotmelder (E) deaktiviert wird, solange keine Anwesenheit eines Teils des lebenden Körpers von dem Anwesenheitsdeteketor (DP) detektiert wird, der diesem Infrarotmelder zugeordnet ist.

3. Verfahren nach Patentanspruch 1, demzufolge jedem Infrarotmelder (E) mehrere Anwesenheitsdetektoren (DP) zugeordnet sind, wobei
- im Verlauf des Aktivierungsschritts ein Infrarotmelder (E) aktiviert wird, wenn alle, ihm zugeordneten Anwesenheitsdetektoren (DP) gleichzeitig die Anwesenheit eines Teils des lebenden Körpers angeben, und
- im Verlauf des Deaktivierungsschritts ein Infrarotmelder (E) deaktiviert wird, sobald einer dieser Anwesenheitsdetektoren (DP) die Anwesenheit eines Teils des lebenden Körpers nicht mehr angibt.

4. Verfahren nach einem der Patentansprüche 1 bis 3, demzufolge von dem Bilderfassungsmittel des Sensors ein Bild erfasst wird, sobald ein Infrarotmelder der Infrarotmeldergruppe aktiviert wird.

5. Verfahren nach einem der Patentansprüche 1 bis 4, demzufolge die Schritte der Aktivierung und Deaktivierung der Infrarotmelder (E) nicht während der Bilderfassung durch das Bilderfassungsmittel des Sensors umgesetzt werden.

6. Venensystemsensor eines Teils eines lebenden Körpers umfassend:
- einen Infrarotmeldergruppe,
- ein Erfassungsmittel zum Erfassen von Bildern dieses, sich gegenüber der Infrarotmeldergruppe befindlichen Venensystems,
und **dadurch gekennzeichnet, dass** es zudem folgendes umfasst:
- eine, sich in Bezug auf das Erfassungsmittel auf derselben Seite wie die Infrarotmelder befindliche Anwesenheitsdetektorgruppe,
wobei jeder Anwesenheitsdetektor wenigstens einem Infrarotmelder und jeder Infrarotmelder wenigstens einem Anwesenheitsdetektor zugeordnet ist, wobei jeder Anwesenheitsdetektor dazu geeignet ist, zu bestimmen, ob ein Teil des lebenden Körpers zwischen dem Infrarotmelder, dem dieser Anwesenheitsdetektor zugeordnet ist, und dem Bilderfassungsmittel anwesend ist,
- Mittel zur Aktivierung wenigstens eines Infrarotmelders, wobei die Aktivierung dann erfolgt, wenn die Anwesenheit eines Teils des lebenden Körpers (CV) von wenigstens einem, dem genannten Infrarotmelder zugeordneten Anwesenheitsdetektor (DP) detektiert wird,
- Mittel zur Deaktivierung wenigstens eines Infrarotmelders, wobei die Deaktivierung des genannten Infrarotmelders (E) im gegenteiligen Fall erfolgt.

7. Sensor nach Patentanspruch 6, **dadurch gekennzeichnet, dass** die Infrarotmelder und die Anwesenheitsdetektoren in Zeilen und Spalten positioniert sind.

8. Sensor nach Patentanspruch 7, **dadurch gekennzeichnet, dass** sich in jeder Zeile und Spalte ein Infrarotmelder und ein Anwesenheitsdetektor abwechseln.

9. Sensor nach Patentanspruch 7, **dadurch gekennzeichnet, dass** jedem Infrarotmelder vier Anwesenheitsdetektoren zugeordnet sind und dass jeder dieser vier Detektoren in einer Himmelsrichtung des genannten Infrarotmelders positioniert ist.

10. Sensor nach einem der Patentansprüche 6 bis 9, **dadurch gekennzeichnet, dass** wenigstens ein Anwesenheitsdetektor, der Anwesenheitsdetektorengruppe ein Schalter ist, der auf einen Lichtstrom reagiert und dadurch, dass der Sensor eine einzige Lichtquelle (S) umfasst, die dazu vorgesehen ist, den wenigstens einen Schalter zu beleuchten.

11. Sensor nach Patentanspruch 10, **dadurch gekennzeichnet, dass** die einzige Lichtquelle (S) so positioniert ist, dass sie entweder den genannten wenigstens einen Schalter durch Reflexion auf einen Teil des lebenden Körpers oder den genannten wenigstens einen Schalter in Abwesenheit eines lebenden Körpers beleuchtet.

## Claims

1. Method for slaving, to the presence of a living body, the activation of a set of infrared emitters of a venous network sensor comprising also an acquisition means located in front of the set of infrared emitters,
the method comprising a step of detecting the presence of the living body,
the method being **characterized in that** the sensor comprises also a set of presence detectors located on the same side that the infrared emitters with respect to the acquisition means, each presence detector being associated with at least one infrared emitter, each infrared emitter being associated with at least one presence detector,
**in that** the method comprises after the step of detecting:
- an activation step during which each infrared emitter (E) is activated if the presence of a part of the living body (CV) is detected by at least one presence detector (DP) that is associated therewith and, in the contrary case:
- a deactivation step during which the said infrared emitter (E) is deactivated, and
**in that** the step of detecting is such that each presence detector determines whether a part of the living body is present between the infrared emitter with which this presence detector is associated and the acquisition means.

2. Method according to claim 1, in which, only one presence detector (DP) being associated with each infrared emitter (E),
- during the activation step, an infrared emitter (E) is activated if the presence of a part of the living body is detected by the presence detector (DP) that is associated therewith, and
- during the deactivation step, an infrared emitter (E) is deactivated as long as the presence of a part of the living body is not detected by the presence detector (DP) that is associated therewith.

3. Method according to claim 1, in which several presence detectors (DP) are associated with each infrared emitter (E),
- during the activation step, an infrared emitter (E) is activated when all the presence detectors (DP) that are associated therewith simultaneously indicate the presence of a part of the living body, and
- during the deactivation step, an infrared emitter (E) is deactivated as soon as one of these presence detectors (DP) no longer indicates the presence of a part of the living body.

4. Method according to one of claims 1 to 3, in which an image is acquired by the image acquisition means of the sensor as soon as an infrared emitter in the set is activated.

5. Method according to one of claims 1 to 4, in which the steps of activation and deactivation of the infrared emitters (E) are not implemented during the acquisition of an image by the image acquisition means of the sensor.

6. Sensor for venous networks in a part of a living body, comprising:
- a set of infrared emitters,
- an image acquisition means of this venous network located in front of the set of infrared emitters,
and **characterized in that** it further comprises:
- a set of presence detectors located on the same side that the infrared emitters with respect to the acquisition means,
each presence detector being associated with at least one infrared emitter, each infrared emitter being associated with at least one presence detector, each presence detector being adapted to determine whether a part of the living body is present between the infrared emitter with which this presence detector is associated and the acquisition means,
- activation means of at least one infrared emitter, activated if the presence of a part of the living body (CV) is detected by at least one presence detector (DP) that is associated therewith,
- deactivation means of at least one infrared emitter activated for said infrared emitter (E) in the contrary case.

7. Sensor according to claim 6, **characterized in that** the infrared emitters and the presence detectors are positioned in row and column.

8. Sensor according to claim 7, **characterized in that** in each row and column, an infrared emitter alternates with a presence detector.

9. Sensor according to claim 7, **characterized in that** four presence detectors are associated with each infrared emitter, and **in that** each of these four presence detectors is positioned at a cardinal point of said infrared emitter.

10. Sensor according to one of claims 6 to 9, **characterized in that** at least one presence detector in the set is a switch reacting to a light flux, and **in that** the sensor comprises a single light source (S) designed to illuminate said at least one switch.

11. Sensor according to claim 10, **characterized in that** the single light source (S) is positioned so as either to illuminate said at least one switch by reflection on a part of the living body, or to illuminate said at least one switch in the absence of a living body.
